# FASCICULE DE BREVET EUROPEEN

(11) **EP 3 548 563 B1**
(45) Date de publication et mention de la délivrance du brevet: **10.08.2022**
(21) Numéro de dépôt: 17811661.2
(22) Date de dépôt: 27.11.2017
(51) Int. Cl.: C09B 67/54, C09B 7/02, A61K 49/00, A23L 5/47

(54) **PROCEDE DE PREPARATION DU CARMIN D'INDIGO**
VERFAHREN ZUR HERSTELLUNG VON INDIGOKARMIN
PROCESS FOR PREPARING INDIGO CARMINE

(30) Priorité: 01.12.2016 FR 1661784
(43) Date de publication de la demande: 09.10.2019
(73) Titulaire: Provepharm Life Solutions, 13013 Marseille (FR)
(72) Inventeur: SAYAH, Babak, 13013 Marseille (FR); QUERU, Stéphane, 13013 Marseille (FR); FERAUD, Michel, 13013 Marseille (FR); LOPEZ, Nicolas, 13013 Marseille (FR)
(74) Mandataire: Corizzi, Valérie
(86) Numéro de dépôt international: PCT/FR2017/053260
(87) Numéro de publication internationale: WO 2018/100277

(56) Documents cités:
- WO-A1-2011/016487
- WO-A1-2014/001050
- WO-A1-2015/114548
- WO-A1-2015/130539
- DE-A1- 2 742 875
- DE-C- 180 097
- US-A1- 2014 155 625
- IQBAL T. SHADI ET AL: "Analysis of the conversion of indigo into indigo carmine dye using SERRS", CHEMICAL COMMUNICATIONS - CHEMCOM., no. 12, 1 janvier 2004 (2004-01-01), page 1436, XP055288156, ISSN: 1359-7345, DOI: 10.1039/b403601h
- FERNANDO AGUILAR: "Scientific Opinion on the re-evaluation of Indigo Carmine (E 132) as a food additive", THE EFSA JOURNAL, vol. 12, no. 7, 1 juillet 2014 (2014-07-01), page 3768, XP055288179, Parma, IT ISSN: 1831-4732, DOI: 10.2903/j.efsa.2014.3768

## Description

La présente invention concerne un nouveau procédé de préparation de la molécule de carmin d'indigo, ce procédé permettant d'atteindre un degré de pureté élevé, tout en étant très simple de mise en oeuvre et présentant des rendements élevés.

### Etat de la technique antérieure

Le carmin d'indigo, également connu sous les noms de 5,5'-indigosulfonate de di-sodium et de 3,3'-dioxo-2,2'-bis-indolyden-5,5'-disulfonate de di-sodium (numéro CAS 860-22-0) est connu pour ses utilisations comme colorant, dans les domaines alimentaire, pharmaceutique et cosmétique, mais aussi dans le domaine de l'impression, de la photographie (R.W. Sabnis, Handbook of Biological Dyes and Stains, 2010, Wiley and Sons, p.239). Dans le domaine alimentaire, l'indigo carmin fait l'objet de spécifications qui sont rapportées dans EFSA Journal 2014 ; 12(7) : 3768. Dans le domaine pharmaceutique il est utilisé notamment pour la coloration des tissus, pour la détection de certaines cellules tumorales. Le 5,5'-indigosulfonate de disodium est généralement obtenu à partir de l'indigo ou 2-(1,3-dihydro-3-oxo-2H-indole-2-ylidène)-1,2-dihydro-3H-indole-3-one (numéro CAS : 482-89-3), suivant une réaction résumée sur le schéma 1 ci-dessous :

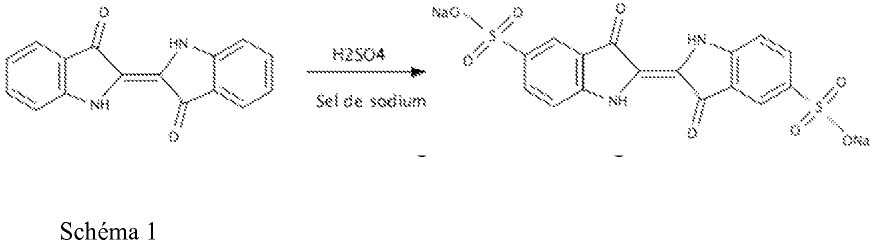

Toutefois, au cours de cette réaction, il se forme habituellement d'autres composés mono-, di- tri- ou tétrasulfonatés, majoritairement sur les positions 5, 5', 7, 7'.

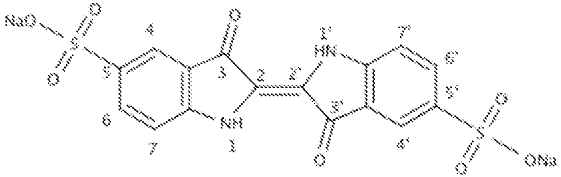

### 5,5'-indigosulfonate de di-sodium

Les isomères disulfonatés sont présents en quantités importantes, et leurs propriétés physico-chimiques sont très proches de celles de l'indigo carmin. La proximité de structure des molécules disulfonatées rend leur séparation fastidieuse. La présence d'isomères disulfonatés présente l'inconvénient, pour les applications médicales ou de diagnostic, d'introduire dans l'organisme du patient des contaminants non désirés. Sung-Hoon Kim et al., Korean J. Anesthesiol. 2011 Nov ; 61(5) : 435-438 ont décrit des cas d'hypotension chez des patients auxquels un indigo carmin contenant des impuretés avait été administré. Ces effets secondaires ont été attribués aux impuretés.

L'objectif de l'invention a donc été de mettre au point un procédé de préparation de 5,5'-indigosulfonate de di-sodium sensiblement dépourvu d'impuretés, et notamment d'impuretés disulfonatées. On a cherché à mettre au point un procédé qui donne accès à un 5,5'-indigosulfonate de di-sodium conforme aux exigences de l'EFSA dans le domaine alimentaire et à la norme ICH dans le domaine pharmaceutique (International conference on harmonisation of technical requirements for registration of pharmaceuticals for human use ; ICH harmonised tripartite guideline - Impurities in new drug substances - Q3A(R2) ; *Step 4* version ; 25 October 2006). La norme ICH spécifie qu'un principe actif ne devrait pas contenir d'impuretés non identifiées en une teneur supérieure ou égale à 0,10% par rapport au principe actif. On a cherché à mettre au point un procédé qui soit rapide, peu coûteux, efficace, avec des rendements élevés, facilement extrapolable à l'échelle industrielle.

### Résumé de l'invention

L'invention concerne un procédé de préparation de 5,5'-indigosulfonate de disodium, le produit de départ étant de l'indigo, ce procédé comprenant les étapes suivantes :
i) l'indigo est soumis à un traitement de sulfonation conduisant à un mélange qui contient de l'acide 5,5'-indigosulfonique,
   ce procédé étant caractérisé en ce que :
   ii) on applique un traitement de réduction au mélange obtenu à l'étape i), et éventuellement une étape de purification, de façon à obtenir une composition comprenant de l'acide leuco-5,5'-indigosulfonique,
   iii) l'acide leuco-5,5'-indigosulfonique est isolé de la composition issue de l'étape ii),
   iv) l'acide leuco-5,5'-indigosulfonique issu de l'étape iii) est oxydé en 5,5'-indigosulfonate de di-sodium.

Selon un mode de réalisation préféré, le traitement de réduction de l'étape i) est mis en oeuvre au moyen de dithionite de sodium en milieu aqueux.

Selon un mode de réalisation préféré, l'acide leuco-5,5'-indigosulfonique précipite dans le milieu réactionnel à l'issue de l'étape ii).

Selon un mode de réalisation préféré, l'étape iii) comporte au moins une étape consistant en :
iiia) une filtration de la composition issue de l'étape ii),
iiib) la récupération du solide retenu sur le filtre.

Avantageusement, l'étape iii) comporte en outre après l'étape iiib) au moins une étape iiic) consistant en un lavage du solide par un milieu aqueux.

Avantageusement encore, l'étape iiic) est appliquée de une à cinq fois.

Selon un mode de réalisation préféré, la composition obtenue à l'issue de l'étape iii) comporte au moins 90% en moles d'acide leuco-5,5'-indigosulfonique par rapport au nombre de moles total de la composition.

Selon un mode de réalisation préféré, l'étape iv) comporte au moins une étape iva) consistant en un traitement par une base en milieu alcoolique en présence d'un agent oxydant.

Avantageusement, l'étape iva) consiste en un traitement par de l'éthanolate de sodium dans de l'éthanol, en présence d'un agent oxydant.

Avantageusement, l'agent oxydant est de l'oxygène gazeux.

Selon un mode de réalisation préféré, le procédé comporte en outre, après l'étape iva), au moins une étape ivb) consistant en un ou plusieurs lavages par un ou plusieurs solvants alcooliques et/ou hydroalcooliques.

L'invention concerne également l'utilisation du procédé défini ci-dessus pour produire une composition comprenant au moins 99,5% de 5,5'-indigosulfonate de disodium, le % étant mesuré par chromatographie en phase liquide à haute pression avec une détection à 290nm.

L'invention concerne encore l'utilisation du procédé tel que défini ci-dessus pour produire une composition dans laquelle aucune impureté n'est présente en quantité supérieure à 0,10%, le % étant mesuré par chromatographie en phase liquide à haute pression avec une détection à 290nm.

L'invention concerne aussi un procédé de fabrication d'un médicament ou d'un produit de diagnostic comprenant du 5,5'-indigosulfonate de di-sodium, ce procédé comprenant la fabrication du 5,5'-indigosulfonate de di-sodium par le procédé décrit ci-dessus et de façon détaillée ci-dessous, et l'introduction du 5,5'-indigosulfonate de di-sodium dans un support pharmaceutiquement acceptable.

L'invention concerne également un procédé de fabrication d'une composition alimentaire comprenant du 5,5'-indigosulfonate de di-sodium, ce procédé comprenant la fabrication du 5,5'-indigosulfonate de di-sodium par le procédé décrit ci-dessus et de façon détaillée ci-dessous, et l'introduction du 5,5'-indigosulfonate de di-sodium dans un support compatible avec une application alimentaire.

Par exemple, ce dernier procédé peut concerner la fabrication d'une composition de colorant alimentaire, ou d'une composition alimentaire colorée.

Les inventeurs ont observé de façon étonnante que l'acide leuco-5,5'-indigosulfonique pouvait être facilement séparé de ses isomères disulfonatés, obtenus par sulfonation de l'indigo et application d'un traitement de réduction, tandis que les mélanges d'acide 5,5'-indigosulfonique et ses isomères disulfonatés, sous forme acide ou sous forme de di-sels de sodium, sont difficiles à séparer.

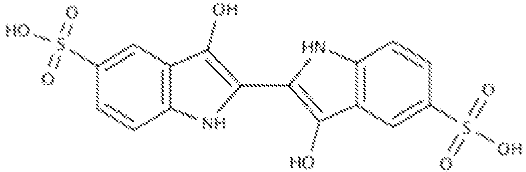

### Acide leuco-5,5'-indigosulfonique

En effet, la réduction a été faite dans un milieu réactionnel dans lequel l'acide leuco-5,5'-indigosulfonique est peu soluble, tandis que ses contaminants, notamment ses isomères disulfonatés, sont solubles dans ce milieu.

Le passage par ces produits intermédiaires à l'état réduit permet donc une séparation efficace, avec des rendements élevés. On récupère ainsi l'acide leuco 5,5'-indigosulfonique. Le produit isolé peut ensuite être soumis à un traitement d'oxydation de façon à former un 5,5'-indigosulfonate de di-sodium présentant un niveau de pureté élevé.

### Description détaillée

### Etape i) : sulfonation de l'indigo

Le produit de départ est l'indigo ou 2-(1,3-dihydro-3-oxo-2H-indole-2-ylidène)-1,2-dihydro-3H-indole-3-one (CAS : 482-89-3). L'indigo est soumis à un traitement de sulfonation suivant le schéma 2 ci-dessous :

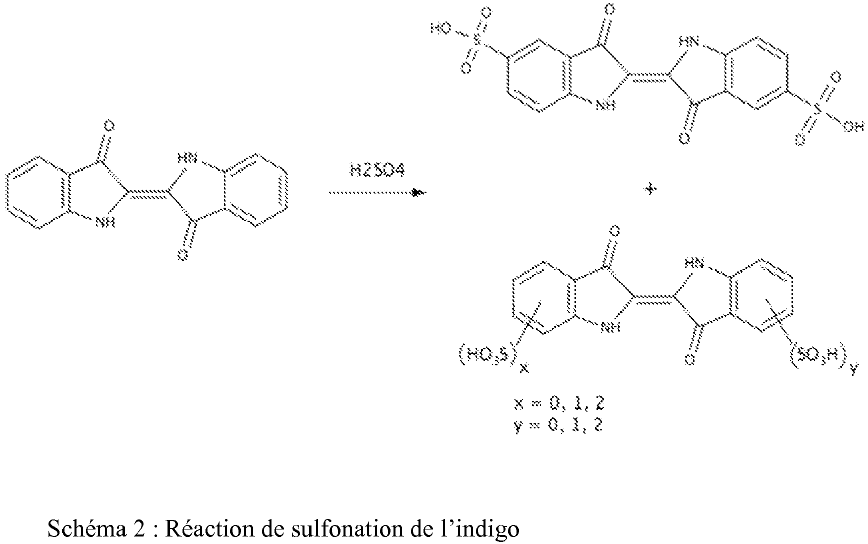

Cette réaction connue est habituellement mise en oeuvre par traitement par de l'acide sulfurique. On peut par exemple se reporter aux publications suivantes : US-647280 ; Tudora Baltac et al., Revista de Chimie (Bucharest, Romania) (2012), 63(6), 618-620 ; Iqbal T. Shadi et al., Chemical Communications (Cambridge, United Kingdom) (2004), (12), 1436-1437 ; Capron, F. (1863), Blues and carmines of indigo: a practical treatise on the fabrication of every commercial product derived from indigo ; Philadelphia: H. C. Baird (https://catalog.hathitrust.org/Record/006218585) ; Vanessa Bianda et al., Bulgarian Journal of Science Education, Volume 22, Number 1, 2013 ; Charles E. Carraher et al., Journal of Macromolecular Science, Volume 15, Issue 5 1981, pages 773-785 ; R.W. Sabnis, Handbook of Biological Dyes and Stains, 2010, Wiley and Sons, p.239.

Toutefois, le procédé de l'invention n'est pas limité au produit de cette réaction et peut s'appliquer à toute composition comprenant de l'acide 5,5'-indigosulfonique et des isomères disulfonatés de l'acide 5,5'-indigosulfonique, quel que soit leur procédé d'obtention.

Lorsque la composition de départ est issue de la sulfonation de l'indigo, elle peut comprendre également, en plus de l'acide 5,5'-indigosulfonique et des isomères disulfonatés : de l'indigo, des dérivés monosulfonatés, trisulfonatés, tétrasulfonatés, ainsi que d'autres produits secondaires de cette réaction. Toutefois, de façon habituelle, ces autres dérivés sont éliminés au moyen d'une méthode connue, telle que par exemple une chromatographie liquide à haute pression (CLHP).

Lorsque la composition de départ est issue de la sulfonation de l'indigo, elle comprend environ de 60 à 95 % de l'acide 5,5'-indigosulfonique et de 5 à 40 % d'isomères disulfonatés de préférence de 75 à 80 % de l'acide 5,5'-indigosulfonique et de 20 à 25 % d'isomères disulfonatés, les pourcentages étant donnés en nombre de moles par rapport à la somme des nombres de moles de l'ensemble des molécules disulfonatées. Toutefois, le procédé de l'invention peut s'appliquer à une composition comprenant un mélange d'isomères en toutes proportions.

Avantageusement, pour une mise en oeuvre dans le procédé de l'invention, on utilise une composition de départ dans laquelle l'acide 5,5'-indigosulfonique et ses isomères disulfonatés représentent au moins 90 % molaire par rapport au nombre de moles total de la composition de départ, encore plus avantageusement au moins 95 %, et encore mieux au moins 98 %.

Par « isomères disulfonatés » de l'acide 5,5'-indigosulfonique, on entend toute molécule présentant la structure de l'indigo, ou 2-(1,3-dihydro-3-oxo-2H-indole-2-ylidène)-1,2-dihydro-3H-indole-3-one, porteuse d'une substitution par une fonction acide sulfonique ou sulfonate sur chacun des groupements indole. L'acide 5,7'-indigosulfonique est un exemple d'isomère disulfonaté de l'acide 5,5'-indigosulfonique.

### Etape ii) : réduction du mélange issu de l'étape i)

Dans une première étape, le mélange issu de l'étape i) comprenant de l'acide 5,5'-indigosulfonique est soumis à un traitement de réduction conduisant à un mélange d'acide leuco-5,5'-indigosulfonique et d'autres composés.

Avantageusement ce traitement de réduction est réalisé en mettant en œuvre du dithionite de sodium comme réactif réducteur. De façon alternative, on pourrait utiliser l'un ou l'autre des agents réducteurs suivants : un mélange d'arsenic sesquisulfide et d'hydrogénosulfite de sodium, du sulfate de fer (II), du zinc, ou on pourrait réaliser une réduction par électrolyse.

De préférence, le traitement de réduction est mis en oeuvre en milieu aqueux.

Avantageusement, le traitement de réduction est mis en oeuvre en appliquant un chauffage à une température allant de 40 à 60°C, avantageusement de 45 à 55°C.

De préférence, la durée totale du traitement est de 1/2h à 6h, avantageusement de 1 h à 4h.

De préférence, après le traitement de réduction, la composition obtenue est purifiée dans des conditions permettant d'augmenter la teneur en acide leuco-5,5'-indigosulfonique. Notamment le milieu est extrait par un solvant organique de façon à éliminer certains contaminants, comme par exemple l'indigo réduit. L'extraction peut être faite par l'acétate d'éthyle, ou par tout autre solvant adapté pour extraire les composants non sulfonatés.

### Etape iii) : séparation de l'acide leuco-5,5'-indigosulfonique

Dans une seconde étape, l'acide leuco-5,5'-indigosulfonique est séparé du milieu réactionnel. Avantageusement, l'acide leuco-5,5'-indigosulfonique est séparé du milieu réactionnel par précipitation de l'acide leuco-5,5'-indigosulfonique, suivie d'une filtration et récupération du solide.

De préférence, l'étape ii) a été mise en oeuvre dans un solvant dans lequel l'isomère acide leuco-5,5'-indigosulfonique précipite. De préférence, l'isomère acide leuco-5,5'-indigosulfonique est séparé à partir d'une composition aqueuse le comprenant. Avantageusement, la séparation est réalisée directement sur le milieu réactionnel issu de l'étape ii), éventuellement après une première purification, telle que par exemple une extraction au moyen d'un solvant organique. De préférence, l'acide leuco-5,5'-indigosulfonique précipite dans le milieu réactionnel à l'issue de l'étape ii).

De façon alternative, si la réaction de l'étape ii) était mise en oeuvre dans un milieu dans lequel l'acide leuco-5,5'-indigosulfonique est soluble, on pourrait ensuite procéder à un ajout d'eau dans le milieu réactionnel, ou à un échange de solvants, de façon à faire précipiter l'acide leuco-5,5'-indigosulfonique.

De préférence, la séparation est réalisée par passage du milieu réactionnel issu de l'étape ii) sur un support de filtration. Par exemple, on peut filtrer le milieu réactionnel issu de l'étape ii) sur un filtre de type : entonnoir filtrant de porosité 3 ou toile filtrante polypropylène multifilaments. Avantageusement, le milieu réactionnel issu de l'étape ii) est filtré à chaud, de préférence à une température allant de 40 à 60°C, avantageusement de 45 à 55°C.

Dans ces conditions, l'isomère acide leuco-5,5'-indigosulfonique, solide, est retenu par le filtre, tandis que les autres composants du milieu réactionnel, solubles dans le milieu réactionnel de l'étape ii), sont entraînés dans la phase aqueuse pendant la filtration.

Avantageusement cette filtration peut être complétée par un traitement consistant à empâter le solide dans un solvant organique, tel que par exemple du THF, et à filtrer la pâte ainsi formée. Cet empâtage permet d'améliorer le degré de pureté de l'acide leuco-5,5'-indigosulfonique.

Selon l'invention, on prévoit avantageusement de compléter l'étape de séparation de l'acide leuco-5,5'-indigosulfonique par une purification supplémentaire telle qu'un lavage à l'eau, par exemple en reprenant la phase solide comprenant l'acide leuco-5,5'-indigosulfonique, dans de l'eau, que l'on porte à une température allant de 40 à 60°C, avantageusement de 45 à 55°C, puis en filtrant la suspension ainsi préparée. Le solide récupéré sur le filtre est l'acide leuco-5,5'-indigosulfonique. Son degré de pureté est amélioré par cette étape de purification additionnelle.

Avantageusement, l'opération de lavage à l'eau de l'acide leuco-5,5'-indigosulfonique est appliquée de une à cinq fois, encore plus avantageusement de deux à quatre fois.

Avantageusement ce lavage peut être complété par un traitement consistant à empâter le solide dans un solvant organique, tel que par exemple du THF, et à filtrer la pâte ainsi formée. Cet empâtage permet d'améliorer le degré de pureté de l'acide leuco-5,5'-indigosulfonique.

De façon avantageuse, les traitements de purification sont répétés jusqu'à obtenir un acide leuco-5,5'-indigosulfonique présentant le niveau de pureté attendu. De préférence, les traitements de purification sont répétés jusqu'à obtenir une composition comprenant au moins 99% molaire d'acide leuco-5,5'-indigosulfonique, par rapport au nombre de moles de la composition.

L'acide leuco-5,5'-indigosulfonique est un intermédiaire essentiel à la mise en oeuvre du procédé de l'invention.

On pourrait avoir recours à d'autres techniques, comme par exemple la chromatographie liquide haute pression (CLHP), pour séparer l'acide leuco-5,5'-indigosulfonique du milieu réactionnel issu de l'étape ii). Toutefois, la filtration présente l'avantage d'être une méthode pratique, efficace, et très facilement extrapolable à l'échelle industrielle.

### Etape iv) : oxydation de l'acide leuco-5,5'-indigosulfonique

Dans une quatrième étape, l'acide leuco-5,5'-indigosulfonique est transformé en 5,5'-indigosulfonate de di-sodium par un traitement d'oxydation approprié, suivant le schéma 3 ci-dessous :

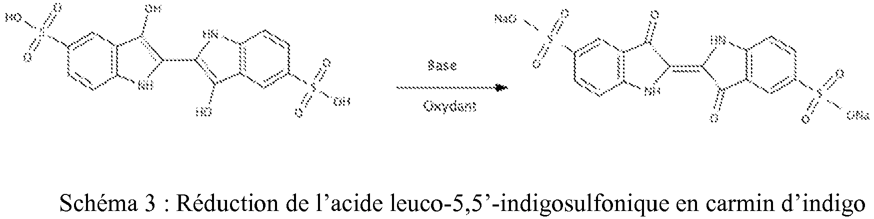

Avantageusement, ce traitement consiste en un traitement par une base en milieu alcoolique en présence d'un agent oxydant. De préférence, l'acide leuco-5,5'-indigosulfonique est traité par de l'alcoolate de sodium en milieu alcool et en présence d'un agent oxydant.

De préférence, l'acide leuco-5,5'-indigosulfonique est traité par de l'éthanolate de sodium en milieu éthanol en présence d'un agent oxydant. De préférence, le traitement est réalisé à chaud, avantageusement à une température allant de 40 à 60°C, encore plus avantageusement de 45 à 55°C. De préférence, l'acide leuco-5,5'-indigosulfonique est traité par 0,5 à 2,5 équivalents molaires d'alcoolate de sodium, de préférence 0,8 à 1,5 équivalents molaires d'alcoolate de sodium, en présence d'un agent oxydant. Encore plus préférentiellement, l'acide leuco-5,5'-indigosulfonique est traité par 0,5 à 2,5 équivalents molaires d'éthanolate de sodium, de préférence 0,8 à 1,5 équivalents molaires d'éthanolate de sodium, en présence d'un agent oxydant. Avantageusement, l'agent oxydant est choisi parmi O₂, H₂O₂, KHSO₅, FeCl₃

De préférence l'agent oxydant est de l'oxygène gazeux, qui peut être utilisé pur ou en mélange avec d'autres gaz, comme par exemple l'oxygène de l'air. De préférence, la durée du traitement est de 0,5 h à 10h, encore plus préférentiellement de 1h à 8h, et avantageusement de 2h à 6h.

De préférence, le traitement de l'étape iv) est suivi d'un ou plusieurs lavages par un ou plusieurs solvants organiques. De préférence, le traitement de l'étape iv) est suivi d'un ou plusieurs lavages par un ou plusieurs solvants alcooliques et/ou hydroalcooliques, notamment un lavage par de l'éthanol et/ou un lavage par du méthanol et/ou un lavage par un mélange d'eau et de méthanol et/ou un lavage par un mélange d'eau et d'éthanol.

### Utilisations :

L'invention a encore pour objet l'utilisation du procédé décrit ci-dessus pour produire un carmin d'indigo ou 5,5'-indigosulfonate de di-sodium présentant un niveau de pureté élevé. En particulier, l'invention concerne l'utilisation du procédé décrit ci-dessus pour produire une composition comprenant au moins 99,5%, encore mieux au moins 99,7%, de carmin d'indigo ou 5,5'-indigosulfonate de di-sodium. Pour l'évaluation de la quantité d'indigo carmin dans la composition de matière, le % est mesuré par chromatographie en phase liquide à haute pression (CLHP) avec une détection à 290nm, sur colonne C18 de 5µm en 150x4.6, avec une élution par gradient tampon phosphate de sodium 10mM (pH 3.0) + 1mM TBACl/MeOH (70/30→10/90).

L'invention concerne l'utilisation du procédé décrit ci-dessus pour produire une composition dans laquelle aucune impureté n'est présente en quantité supérieure à 0,10%, encore mieux aucune impureté n'est présente en quantité supérieure à 0,05%. L'évaluation de la quantité des impuretés est réalisée au moyen de la même méthode que celle mise en oeuvre pour l'évaluation de la quantité d'indigo carmin.

L'invention a encore pour objet l'utilisation du procédé décrit ci-dessus pour produire un médicament ou un produit de diagnostic comprenant du carmin d'indigo ou 5,5'-indigosulfonate de di-sodium. Ledit médicament ou produit de diagnostic peut être sous toute forme adaptée à son utilisation dans ces applications. En particulier, on peut mentionner : sous forme de comprimé ou de gélule comprenant de 1 à 1000 mg de carmin d'indigo ; sous forme de solution aqueuse comprenant du carmin d'indigo à une concentration allant de 0.1% à 10% massique. De telles compositions comprennent, outre le carmin d'indigo, des excipients bien connus de l'homme du métier, tels que par exemple de l'acide citrique et/ou des citrates, un tampon phosphate, des polymères, des dérivés de la cellulose, des lipides. Des formulations comprenant de l'indigo carmin sont illustrées notamment dans WO2011107945 et dans WO2010018723. Dans les applications médicales et de diagnostic, le carmin d'indigo obtenu par le procédé de l'invention présente l'avantage d'une pureté élevée, ce qui évite d'introduire dans l'organisme des matériaux sans utilité pour l'application.

L'invention a encore pour objet l'utilisation du procédé décrit ci-dessus pour produire un colorant alimentaire comprenant du carmin d'indigo ou 5,5'-indigosulfonate de di-sodium. Ledit colorant alimentaire peut être sous toute forme adaptée à son utilisation dans ces applications, notamment sous forme de poudre ou de solution aqueuse. Le niveau de pureté élevé du carmin d'indigo obtenu par le procédé de l'invention permet de garantir son innocuité.

L'efficacité du procédé de l'invention permet d'accéder à un produit présentant des coûts réduits.

### Partie expérimentale :

### I- Matériaux et méthodes :

1- Matières premières et équipement :
   L'indigo a été acheté auprès de la société Fisher Scientific sous la référence commerciale 21213. Pureté : 94,8 % selon spécifications du fournisseur.
   Toile (filtration) : référence commerciale V-05-6-475 K disponible auprès de la société SEFAR.
   Filtre Nutsche : diamètre 280 mm, 35 L, disponible auprès de la société BUCHI.
2- Méthodes d'analyse :
   Appareil : Agilent 1100 ^{®}
   Colonne : KROMASIL C18 ^{®} 150 × 4.6-5µm
   Détection : à 290 nm
   Concentration de l'échantillon : 1000 ppm
   Solvant de dissolution de l'échantillon : H₂O/MeOH 90/10
   Solvant d'élution : Tampon Sodium phosphate 10 mM (pH 3.0) + 1mM TBACl/MeOH

### II- Protocoles :

### 1- Transformation de l'indigo en acide 5,5'-indigosulfonique :

Dans un réacteur de 10 litres, on charge 1,5 kg d'indigo (5,72 moles) auquel on ajoute, sous agitation, et à température ambiante, 6 litres d'une solution aqueuse de H₂SO₄ de concentration 96%. On porte le mélange à 70°C pendant 3 heures, toujours sous agitation. Le mélange est ensuite refroidi à 5°C. On ajoute le mélange lentement dans un réacteur de 60 litres contenant 15 litres d'une eau refroidie à 5°C. L'addition du mélange est réalisée en 1h15mn. On ajoute au mélange 90 ml d'octanol et on porte le mélange à 50°C. On obtient un solide en suspension dans le milieu réactionnel qui est un mélange comprenant de l'acide 5,5'-indigosulfonique.

### 2- Réduction du mélange comprenant l'acide 5,5'-indigosulfonique :

On prépare une solution aqueuse de dithionite de sodium à partir de 15 litres d'eau et de 3,51 kg de solution de dithionite de sodium à 85%. La solution aqueuse ainsi préparée est placée dans une ampoule de coulée sous azote. Dans le mélange réactionnel issu de l'étape 1- on ajoute la solution aqueuse de dithionite de sodium, sous agitation, en maintenant le milieu à 50°C. L'addition de la solution aqueuse de dithionite de sodium est réalisée en 1h. Le mélange est ensuite maintenu sous agitation à 50°C pendant environ 1h. On ajoute ensuite au mélange 7,5 litres d'acétate d'éthyle et on maintient le mélange sous agitation à 50°C pendant 20mn. Le milieu est toujours sous forme d'une suspension comprenant de l'acide leuco-5,5'-indigosulfonique.

### 3- Séparation de l'acide leuco-5,5'-indigosulfonique :

Le milieu réactionnel issu de l'étape 2- est ensuite filtré sur un filtre Nutsche, équipé d'une toile V-05-6-475 K.

Le solide resté sur le filtre est empâté avec 4,5 litres de tétrahydrofurane (THF) puis le liquide est filtré.

### 4- Lavage de l'acide leuco-5,5'-indigosulfonique :

Le produit resté sur le filtre à l'issue de l'étape 3- est repris dans 30 litres d'eau, dans un réacteur de 60 litres. L'ensemble est chauffé à 50°C pendant 30 mn puis filtré sur le filtre Nutsche, équipé d'une toile V-05-6-475 K.

Le solide resté sur le filtre est empâté avec 4,5 litres de tétrahydrofurane (THF) puis le liquide est filtré.

Ce lavage est répété de façon à aboutir à un total de trois lavages dans les mêmes conditions. On obtient 1,8 kg d'un produit humide. Masse sèche estimée : 1,3 kg.

### 5- Transformation de l'acide leuco-5,5'-indigosulfonate de di-sodium en 5,5'-indigosulfonate de di-sodium

Dans un réacteur de 60 litres, on met en suspension les 1,8 kg de produit obtenus à l'étape 4- dans 26 litres d'éthanol. On ajoute 1143 ml d'une solution à 21% massique d'éthanolate de sodium (EtONa) dans de l'éthanol. On porte le mélange à 50°C et on fait buller de l'air dans le milieu réactionnel pendant 4h, en le maintenant à 50°C.

Au bout de ce temps, le milieu réactionnel est filtré sur fritté, la filtration étant suivie d'un lavage par 5,2 litres d'éthanol.

### 6- Lavage du 5,5'-indigosulfonate de di-sodium

Le solide humide est repris dans 26 litres de méthanol, l'ensemble est chauffé pendant 30 mn à 40°C puis filtré sur fritté, la filtration étant suivie d'un lavage par 5,2 litres de méthanol.

Ce lavage est répété une fois.

Ensuite, le solide humide est repris dans un mélange de 13 litres d'eau et 13 litres de méthanol, l'ensemble est chauffé pendant 30 mn à 60°C, puis filtré sur fritté, la filtration étant suivie d'un lavage par 3,9 litres de méthanol.

Le solide est séché en étuve ventilée.

On obtient 1,05 kg de 5,5'-indigosulfonate de di-sodium, le rendement est de 39% par rapport à la quantité totale d'indigo engagée dans le procédé.

### III- Résultats :

Le produit obtenu est analysé par HPLC au moyen de la méthode décrite ci-dessus. La pureté est évaluée de 99,85 à 99,95%, en fonction des essais (3 essais réalisés).

On constate que le procédé de l'invention permet d'obtenir un produit de pureté élevée, avec de bons rendements, en appliquant un protocole simple, peu coûteux, facilement extrapolable à grande échelle, et avec des résultats reproductibles du point de vue de la qualité (pureté) du produit obtenu.

## Revendications

1. Procédé de préparation de 5,5'-indigosulfonate de di-sodium, le produit de départ étant de l'indigo, ce procédé comprenant les étapes suivantes :
i) l'indigo est soumis à un traitement de sulfonation conduisant à un mélange qui contient de l'acide 5,5'-indigosulfonique,
ce procédé étant **caractérisé en ce que** :
ii) on applique un traitement de réduction au mélange obtenu à l'étape i), et éventuellement une étape de purification, de façon à obtenir une composition comprenant de l'acide leuco-5,5'-indigosulfonique,
iii) l'acide leuco-5,5'-indigosulfonique est isolé de la composition issue de l'étape ii),
iv) l'acide leuco-5,5'-indigosulfonique issu de l'étape iii) est oxydé en 5,5'-indigosulfonate de di-sodium.

2. Procédé selon la revendication 1, dans lequel le traitement de réduction de l'étape ii) est mis en oeuvre au moyen de dithionite de sodium en milieu aqueux.

3. Procédé selon la revendication 1 ou selon la revendication 2 dans lequel l'acide leuco-5,5'-indigosulfonique précipite dans le milieu réactionnel à l'issue de l'étape ii).

4. Procédé selon l'une quelconque des revendications 1 à 3 dans lequel l'étape iii) comporte au moins une étape consistant en :
iiia) une filtration de la composition issue de l'étape ii),
iiib) la récupération du solide retenu sur le filtre.

5. Procédé selon la revendication 4 dans lequel l'étape iii) comporte en outre après l'étape iiib) au moins une étape iiic) consistant en un lavage du solide par un milieu aqueux.

6. Procédé selon la revendication 5, dans lequel l'étape iiic) est appliquée de une à cinq fois.

7. Procédé selon l'une quelconque des revendications 1 à 6, dans lequel la composition obtenue à l'issue de l'étape iii) comporte au moins 90% en moles d'acide leuco-5,5'-indigosulfonique par rapport au nombre de moles total de la composition.

8. Procédé selon l'une quelconque des revendications 1 à 7 dans lequel l'étape iv) comporte au moins une étape iva) consistant en un traitement par une base en milieu alcoolique en présence d'un agent oxydant.

9. Procédé selon la revendication 8, dans lequel l'étape iva) consiste en un traitement par de l'éthanolate de sodium dans de l'éthanol, en présence d'un agent oxydant.

10. Procédé selon la revendication 8 ou selon la revendication 9, dans lequel l'agent oxydant est de l'oxygène gazeux.

11. Procédé selon l'une quelconque des revendications 8 à 10, qui comporte en outre, après l'étape iva), au moins une étape ivb) consistant en un ou plusieurs lavages par un ou plusieurs solvants alcooliques et/ou hydroalcooliques.

12. Utilisation du procédé selon l'une quelconque des revendications 1 à 11 pour produire une composition comprenant au moins 99,5% de 5,5'-indigosulfonate de disodium, le % étant mesuré par chromatographie en phase liquide à haute pression avec une détection à 290nm.

13. Utilisation du procédé selon l'une quelconque des revendications 1 à 11 pour produire une composition dans laquelle aucune impureté n'est présente en quantité supérieure à 0,10%, le % étant mesuré par chromatographie en phase liquide à haute pression avec une détection à 290nm.

14. Procédé de fabrication d'un médicament ou d'un produit de diagnostic comprenant du 5,5'-indigosulfonate de di-sodium, ce procédé comprenant la fabrication du 5,5'-indigosulfonate de di-sodium par le procédé selon l'une quelconque des revendications 1 à 11 et l'introduction du 5,5'-indigosulfonate de disodium dans un support pharmaceutiquement acceptable.

15. Procédé de fabrication d'une composition alimentaire comprenant du 5,5'-indigosulfonate de di-sodium, ce procédé comprenant la fabrication du 5,5'-indigosulfonate de di-sodium par le procédé selon l'une quelconque des revendications 1 à 11 et l'introduction du 5,5'-indigosulfonate de di-sodium dans un support compatible avec une application alimentaire.

## Patentansprüche

1. Verfahren zur Herstellung von Dinatrium-5,5'-indigosulfonat, wobei das Ausgangsmaterial Indigo ist, wobei das Verfahren die folgenden Schritte umfasst:
i) Indigo wird einer Sulfonierungsbehandlung unterzogen, die zu einem Gemisch führt, das 5,5'-Indigosulfonsäure enthält,
wobei das Verfahren **dadurch gekennzeichnet ist, dass**:
ii) man auf das in Schritt i) erhaltene Gemisch eine Reduktionsbehandlung und gegebenenfalls einen Reinigungsschritt anwendet, um eine Zusammensetzung zu erhalten, die Leuco-5,5'-indigosulfonsäure umfasst,
iii) die Leuco-5,5'-indigosulfonsäure aus der aus Schritt ii) erhaltenen Zusammensetzung isoliert wird,
iv) die aus Schritt iii) erhaltene Leuko-5,5'-indigosulfonsäure zu Dinatrium-5,5'-indigosulfonat oxidiert wird.

2. Verfahren nach Anspruch 1, wobei die Reduktionsbehandlung von Schritt ii) mithilfe von Natriumdithionit in wässrigem Medium durchgeführt wird.

3. Verfahren nach Anspruch 1 oder nach Anspruch 2, wobei die Leuko-5,5'-indigosulfonsäure am Ende von Schritt ii) im Reaktionsmedium ausfällt.

4. Verfahren nach einem der Ansprüche 1 bis 3, wobei Schritt iii) mindestens einen Schritt umfasst, bestehend aus:
iiia) einer Filtration der aus Schritt ii) erhaltenen Zusammensetzung,
iiib) der Rückgewinnung des auf dem Filter zurückgehaltenen Feststoffs.

5. Verfahren nach Anspruch 4, wobei Schritt iii) außerdem nach Schritt iiib) mindestens einen Schritt iiic) umfasst, der aus dem Waschen des Feststoffs mit einem wässrigen Medium besteht.

6. Verfahren nach Anspruch 5, wobei Schritt iiic) ein bis fünf Mal angewendet wird.

7. Verfahren nach einem der Ansprüche 1 bis 6, wobei die am Ende von Schritt iii) erhaltene Zusammensetzung mindestens 90 Mol-% Leuko-5,5'-indigosulfonsäure, bezogen auf die Gesamtanzahl der Mole der Zusammensetzung, enthält.

8. Verfahren nach einem der Ansprüche 1 bis 7, wobei Schritt iv) mindestens einen Schritt iva) umfasst, der aus einer Behandlung mit einer Base in alkoholischem Medium in Gegenwart eines Oxidationsmittels besteht.

9. Verfahren nach Anspruch 8, wobei Schritt iva) aus einer Behandlung mit Natriumethanolat in Ethanol in Gegenwart eines Oxidationsmittels besteht.

10. Verfahren nach Anspruch 8 oder nach Anspruch 9, wobei das Oxidationsmittel gasförmiger Sauerstoff ist.

11. Verfahren nach einem der Ansprüche 8 bis 10, das außerdem nach Schritt iva) mindestens einen Schritt ivb) umfasst, der aus ein- oder mehrmaligem Waschen mit einem oder mehreren alkoholischen und/oder wässrig-alkoholischen Lösungsmitteln besteht.

12. Verwendung des Verfahrens nach einem der Ansprüche 1 bis 11 zur Herstellung einer Zusammensetzung, die mindestens 99,5 % Dinatrium-5,5'-indigosulfonat umfasst, wobei die % durch Hochdruckflüssigkeitschromatographie mit Nachweis bei 290 nm gemessen werden.

13. Verwendung des Verfahrens nach einem der Ansprüche 1 bis 11 zur Herstellung einer Zusammensetzung, in der keine Verunreinigung in einer Menge von mehr als 0,10 % vorhanden ist, wobei die % durch Hochdruckflüssigkeitschromatographie mit Nachweis bei 290 nm gemessen werden.

14. Verfahren zur Herstellung eines Dinatrium-5,5'-indigosulfonat umfassenden Medikaments oder Diagnostik-Produkts, wobei das Verfahren die Herstellung von Dinatrium-5,5'-indigosulfonat durch das Verfahren nach einem der Ansprüche 1 bis 11 und das Einbringen des Dinatrium-5,5'-indigosulfonats in einen pharmazeutisch annehmbaren Träger umfasst.

15. Verfahren zur Herstellung einer Dinatrium-5,5'-indigosulfonat umfassenden Lebensmittelzusammensetzung, wobei das Verfahren die Herstellung von Dinatrium-5,5'-indigosulfonat durch das Verfahren nach einem der Ansprüche 1 bis 11 und das Einbringen des Dinatrium-5,5'-indigosulfonats in einen mit einer Lebensmittelanwendung kompatiblen Träger umfasst.

## Claims

1. A process for preparing disodium 5,5'-indigosulfonate, the starting material being indigo, this process comprising the following steps:
i) indigo is subjected to a sulfonation treatment leading to a mixture which contains 5,5'-indigosulfonic acid,
this process being **characterized in that**:
ii) the mixture obtained in step i) is subjected to a reduction treatment, and optionally a purification step, so as to obtain a composition comprising leuco-5,5'-indigosulfonic acid,
iii) the leuco-5,5'-indigosulfonic acid is isolated from the composition derived from step ii),
iv) the leuco-5,5'-indigosulfonic acid derived from step iii) is oxidized to disodium 5,5'-indigosulfonate.

2. The process as claimed in claim 1, in which the reduction treatment of step ii) is performed using sodium dithionite in aqueous medium.

3. The process as claimed in claim 1 or as claimed in claim 2, in which the leuco-5,5'-indigosulfonic acid precipitates in the reaction medium on conclusion of step ii).

4. The process as claimed in any one of claims 1 to 3, in which step iii) includes at least one step consisting in:
iiia) filtration of the composition derived from step ii),
iiib) recovery of the solid retained on the filter.

5. The process as claimed in claim 4, in which step iii) also includes, after step iiib), at least one step iiic) consisting in washing the solid with an aqueous medium.

6. The process as claimed in claim 5, in which step iiic) is applied from one to five times.

7. The process as claimed in any one of claims 1 to 6, in which the composition obtained on conclusion of step iii) includes at least 90 mol% of leuco-5,5'-indigosulfonic acid relative to the total number of moles of the composition.

8. The process as claimed in any one of claims 1 to 7, in which step iv) includes at least one step iva) consisting of a treatment with a base in alcoholic medium in the presence of an oxidizing agent.

9. The process as claimed in claim 8, in which step iva) consists of a treatment with sodium ethoxide in ethanol, in the presence of an oxidizing agent.

10. The process as claimed in claim 8 or as claimed in claim 9, in which the oxidizing agent is gaseous oxygen.

11. The process as claimed in any one of claims 8 to 10, which also includes, after step iva), at least one step ivb) consisting of one or more washes with one or more alcoholic and/or aqueous-alcoholic solvents.

12. The use of the process as claimed in any one of claims 1 to 11 for producing a composition comprising at least 99.5% of disodium 5,5'-indigosulfonate, the percentage being measured by high-pressure liquid chromatography with detection at 290 nm.

13. The use of the process as claimed in any one of claims 1 to 11 for producing a composition in which no impurity is present in an amount of greater than 0.10%, the percentage being measured by high-pressure liquid chromatography with detection at 290 nm.

14. A process for manufacturing a medicament or a diagnostic product comprising disodium 5,5'-indigosulfonate, this process comprising the manufacture of disodium 5,5'-indigosulfonate via the process as claimed in any one of claims 1 to 11 and the introduction of the disodium 5,5'-indigosulfonate into a pharmaceutically acceptable support.

15. A process for manufacturing a food composition comprising disodium 5,5'-indigosulfonate, this process comprising the manufacture of disodium 5,5'-indigosulfonate via the process as claimed in any one of claims 1 to 11 and the introduction of the disodium 5,5'-indigosulfonate into a support that is compatible with a food application.
